(19) Europäisches Patentamt

European Patent Office

Office européen des brevets

(11) **EP 0 776 659 B1**

(12) **FASCICULE DE BREVET EUROPEEN**

(45) Date de publication et mention
de la délivrance du brevet:
**10.04.2002 Bulletin 2002/15**

(51) Int Cl.⁷: **A61K 9/02**, A61M 31/00,
A61K 9/20

(21) Numéro de dépôt: **96402309.7**

(22) Date de dépôt: **30.10.1996**

(54) **Dispositif de délivrance de médicaments par voie intravaginale**

Intravaginale Freisetzungsvorrichtung für Medikamente

Delivery device for medicaments by the intravaginal route

(84) Etats contractants désignés:
**CH DE FR GB IT LI NL**

(30) Priorité: **01.11.1995 GB 9522403**

(43) Date de publication de la demande:
**04.06.1997 Bulletin 1997/23**

(73) Titulaire: **Aventis Pharma S.A.**
**92160 Antony (FR)**

(72) Inventeur: **Nabahi, Shohré**
**Watchfield, Swindon SN6 8TE (GB)**

(56) Documents cités:
**EP-A- 0 014 514         EP-A- 0 537 559**
**WO-A-95/00199           US-A- 3 545 439**

- **ADVANCED DRUG DELIVERY REVIEWS, vol. 8,
  1992, pages 341-366, XP000613751 JULIE L.
  RICHARDSON ET AL.: "(d) Routes of Delivery:
  Case Studies"**
- **JOURNAL OF OBSTETRICS AND
  GYNAECOLOGY, vol. 4, no. suppl. 1, 1984, pages
  s11-s15, XP000613649 E.M.COUTINHO: "The
  vaginal pill and other new methods of
  contraception"**
- **CONTRACEPTION, vol. 33, no. 1, 1986, pages
  7-22, XP000613650 EGON DICZFALUSY: "New
  developments in oral, injectable and implantable
  contraceptives, vaginal rings and intrauterine
  devices"**
- **Isopropyl myristate, Handbook of
  pharmaceutical excipients, p. 148, Washington,
  American Pharmaceutical Association, 1986.**
- **Isopropylmyristate, HP Fiedler, Lexikon der
  Hilfsstoffe für Pharmazie, Kosmetik und
  angrenzende Gebiete, Aulendorf, Editio Cantor
  Verlag, 1989, p. 661-662.**

**Description**

La présente invention concerne des dispositifs de délivrance de médicaments par voie intravaginale. En particulier, elle concerne de tels dispositifs convenant à l'administration d'une quantité thérapeutique d'un oestrogène et/ou d'un progestogène à une vitesse sensiblement constante sur une période de temps prolongée dans, par exemple, le traitement du déficit en oestrogène.

Le 17β-oestradiol (E2) est l'oestrogène principal produit par l'ovaire pré-ménopausique fonctionnel pendant chaque cycle menstruel. Avec le processus de vieillissement, l'ovulation devient moins fréquente, ce qui conduit à une production réduite de E2. A la ménopause, la production de E2 diminue de façon dramatique jusqu'à moins de 20 µg par jour. Le déficit en oestrogène peut également se produire chez la femme préménopausée par suite d'une maladie, d'une blessure traumatique ou d'oophorectomie.

Les oestrogènes, en particulier le 17β-oestradiol, sont largement utilisés pour le traitement du déficit en oestrogène, le but étant de remplacer les oestrogènes endogènes perdus en raison d'une défaillance ovarienne. Parmi les oestrogènes naturels, le 17β-oestradiol (E2) possède le meilleur profil thérapeutique pour la thérapie substitutive d'oestrogène (ERT - Oestrogen Replacement Therapy) et la thérapie hormonale substitutive (HRT - Hormone Replacement Therapy) et il s'est avéré avoir une fréquence d'effets secondaires particulièrement faible. Particulièrement, le 17β-oestradiol ne stimule pas la synthèse du facteur de coagulation et il n'est pas associé à un risque accru de thrombose veineuse. Le 17β-oestradiol et ses esters semi-synthétiques sont par conséquent largement utilisés dans le traitement des troubles associés au déficit en oestrogène, en particulier des symptômes ménopausiques ou post-ménopausiques.

Par voie orale, les oestrogènes ne sont pas particulièrement bien absorbés, bien que l'on puisse obtenir des taux systémiques raisonnables après micronisation. L'administration par voie orale est suivie principalement par l'absorption par le tractus gastro-intestinal d'où le flux sanguin est vers le foie. En général, les oestrogènes ont un premier passage élevé et environ 70 % est métabolisé pendant le premier passage dans le foie. Le résultat est que la thérapie à l'oestrogène par voie orale conduit à la formation d'oestrone, un oestrogène moins puissant, qui a une influence sur le profil du lipide sanguin. Cela veut dire également qu'il y a une réduction d'environ 30 % dans la disponibilité d'oestrogènes due à l'effet du premier passage.

Un autre inconvénient associé à l'administration de l'oestrogène par voie orale est qu'il implique des doses de bolus- conduisant à des taux d'oestrogène trop élevés après chaque dose et à la nécessité de doser fréquemment.

Les alternatives à l'administration par voie orale incluent les systèmes de délivrance de médicaments par voie transdermique et l'implantation sous-cutanée de pellets à oestrogène. Les systèmes de délivrance de médicaments par voie transdermique ne sont pas susceptibles de produire les taux d'oestrogène nécessaires pour une thérapie de longue durée et doivent être remplacés tous les deux ou trois jours. Les implants sous-cutanés nécessitent une insertion et un enlèvement chirurgicaux et sont difficiles à enlever si des effets secondaires dangereux se développent. Par ailleurs, aucune de ces méthodes n'est susceptible de produire les taux d'oestrogène constants nécessaires pour le traitement d'un déficit en oestrogène sur une longue période de temps.

L'administration d'oestrogène par voie vaginale évite un certain nombre de problèmes associés à la délivrance par voie orale. Il est bien connu que les stéroïdes en général, y compris les oestrogènes, sont absorbés efficacement et rapidement par la muqueuse vaginale. Par ailleurs, la délivrance d'oestrogène dans le vagin est analogue à la sécrétion d'oestrogène dans la circulation sanguine par l'ovaire et évite le métabolisme de premier passage non souhaitable par le foie.

L'oestrogène peut être administré par voie intravaginale à l'aide de crèmes, de solutions ou de comprimés. Toutefois, comme pour l'administration par voie orale, ceux-ci conduisent à la délivrance par bolus plutôt que des taux durables d'oestrogène et nécessitent une application répétée. Pour réaliser une libération prolongée, contrôlée d'oestrogène sur une période de plusieurs mois, un dispositif intravaginal, commodément sous la forme d'un anneau, s'est avéré le plus efficace. Un avantage particulier associé à l'utilisation d'anneaux vaginaux est que ces derniers peuvent être auto-insérés dans le vagin et en être retirés.

Il existe trois type principaux de conception d'anneau. Le premier est l'anneau de matrice, appelé également anneau homogène, dans lequel le principe actif est distribué de façon homogène dans un système élastomère hydrophobe. Dans cette conception, un chargement initial élevé est possible, ce qui conduit à une dose initiale élevée suivie d'une baisse progressive de la vitesse de libération du médicament. La vitesse de libération est exprimée par l'équation donnée ci-dessous (Chien 1985) :

$$Q/t^{\frac{1}{2}} = (1\ A\ Cp.Dp)^{\frac{1}{2}}$$

dans laquelle :

Q =     la quantité d'agent actif libérée par l'unité de surface
A =     la concentration d'agent actif dans la matrice
Cp =    la solubilité d'agent actif dans la matrice siliconée
Dp =    coefficient de diffusion d'agent actif dans la matrice siliconée
t =     temps

La vitesse de libération de l'actif exprimée par cette équation n'est pas d'ordre zéro. Cette conception d'anneau n'est par conséquent pas capable de maintenir une délivrance constante du principe actif sur une période de temps prolongée.

Le deuxième type de conception d'anneau est la conception coeur dans laquelle le principe actif est dispersé de façon homogène dans un coeur élastomère entouré par une membrane contrôlant la vitesse. La vitesse de libération du médicament à partir d'un anneau de ce type peut être exprimée par l'équation suivante (Crank 1967) :

$$R = \frac{2\pi Cs.D.L.}{\ln(b/a)}$$

dans laquelle :

R =     la vitesse de libération
$\pi$ =     3,14
Cs =    solubilité d'agent actif à saturation dans la matrice de polymère
D =     coefficient de diffusion d'agent actif dans la matrice de polymère
b =     diamètre de la gaine
a =     diamètre du coeur
L =     longueur du coeur

La délivrance de doses dans la gamme de 50 à 100 $\mu$g par jour de E2 à partir d'un anneau de ce type nécessite un coeur de grand diamètre utilisé en combinaison avec une membrane particulièrement mince. Les membranes de l'ordre de 0,5 à 1 mm nécessaires pour obtenir une telle dose sont très susceptibles au déchirement pendant la fabrication et pendant l'utilisation et sont également difficiles à fabriquer à l'aide des méthodes de fabrication actuelles.

Le troisième type d'anneau est la conception coque dans laquelle le médicament est contenu dans une bande étroite entre un coeur élastomère hydrophobe central non médicamenteux et une gaine élastomère hydrophobe non médicamenteuse externe étroite. La gaine externe agit comme membrane de contrôle de vitesse. Cette conception d'anneau est difficile à fabriquer et n'est pas capable de délivrer les doses thérapeutiques d'oestrogène nécessaires dans la thérapie hormonale substitutive.

Stumpf et al. (Journal of Clinical Endocrinology and Metabolism 54(1): 208-210 (1982)) décrivent des anneaux vaginaux de polysiloxane pour la délivrance du 17$\beta$-oestradiol. Les anneaux standard d'une conception coque ayant une couche centrale contenant l'estradiol se sont avérés être inefficaces pour assurer une délivrance thérapeutique de 17$\beta$-oestradiol sur une période de temps importante. Des anneaux de conception homogène ont également été étudiés. Ces derniers contenaient 400 mg de 17$\beta$-oestradiol distribués de façon homogène dans tout un anneau ayant une surface de 22 cm$^2$ et une surface de section transversale de 48 mm$^2$. Bien que Stumpf suggère que de tels anneaux sont capables de générer des taux circulants de 17$\beta$-oestradiol dans la gamme physiologique pendant au moins trois mois, les taux de E2 rapportés sont en fait trop élevés pour une utilisation en thérapie hormonale substitutive. Par ailleurs, ces anneaux sont caractérisés par un jet initial de libération de stéroïdes particulièrement élevés pendant le premier mois d'utilisation, suivi d'une baisse progressive du taux de libération.

US-3,545,439 divulgue un anneau vaginal composé d'une matrice de poly-organosiloxane renfermant un progesterone, libéré avec une cinétique d'ordre zéro.

Il existe donc un besoin pour des dispositifs de délivrance de médicaments par voie vaginale améliorés susceptibles de libérer des oestrogènes, en particulier le 17$\beta$-oestradiol, selon un schéma sensiblement d'ordre zéro de façon quotidienne sur une période de plusieurs mois.

Des femmes ayant subi une hystérectomie avec ou sans oophorectomie ne présentent aucun risque de prolifération endométriale et peuvent recevoir le 17$\beta$-oestradiol sans opposition. Toutefois, pour les femmes ayant un utérus intact, la thérapie par le 17$\beta$-oestradiol peut être convenablement combinée avec un progestogène pour réduire le risque de carcinome endométriel.

Il a maintenant été trouvé, de façon surprenante, que l'incorporation d'un ester d'acide gras dans un dispositif de délivrance de médicaments par voie intravaginale est capable de produire les vitesses de libération d'oestrogène et/ou de progestogène désirées sur plusieurs mois.

Ainsi, examinée à partir d'un aspect, l'invention fournit un dispositif de délivrance de médicaments par voie intravaginale contenant un oestrogène et/ou un progestogène dans une matrice de polymère, caractérisée en ce que la matrice de poly organosiloxane comprend en outre au moins un ester d'acide gras, de préférence contenant de 2 à 20 atomes de carbone. De préférence, la matrice de polymère est entourée d'une membrane contrôlant la vitesse.

Sans vouloir être lié par des considérations théoriques, on pense que l'ester d'acide gras sert à augmenter l'hydrophilicité de la matrice de polymère, augmentant ainsi la solubilité de l'oestrogène dans la matrice et augmentant ainsi la vitesse de libération d'oestrogène du dispositif. Cela permet la délivrance des quantités thérapeutiques durables d'oestrogène sur de longues périodes de temps, comme c'est nécessaire dans la thérapie substitutive à l'oestrogène (ERT) et la thérapie hormonale substitutive (HRT).

Les esters d'acides gras préférés pour une utilisation dans le dispositif selon l'invention incluent ceux formés à partir d'acides contenant de 2 à 20 atomes de carbone, notamment des acides gras à longue chaîne, par ex. l'acide caproïque, laurique, myristique, oléique, linoléique, adipique et lanolique. On préfère notamment les esters formés à partir de l'acide myristique. Les alcools pouvant être utilisés dans la formation des esters incluent ceux contenant de 2 à 20 atomes de carbone, notamment ceux contenant de 2 à 4 atomes de carbone, par ex. le propanol, en particulier l'isopropanol. Un ester d'acide gras particulièrement préféré est le myristate d'isopropyle (IPM).

La matrice de polymère comprends un ou plusieurs organopolysiloxanes. Les élastomères préférés incluent les organopolysiloxanes à extrémité hydroxyle du type RTV (vulcanisation à température ambiante) qui se durcissent en élastomères à température ambiante suivant l'addition d'agents réticulants en présence de catalyseurs de durcissement. Des agents réticulants et des catalyseurs de durcissement appropriés sont connus dans la technique. Un catalyseur de durcissement typique est l'octoate stanneux. La température de durcissement et le temps de durcissement: peuvent varier dans de large gammes et sont fonction de l'élastomère particulier utilisé. La température de durcissement peut varier entre la température ambiante et 150° C, mais elle est de préférence comprise dans la gamme de 60 à 90° C. Le temps de durcissement peut varier entre quelques secondes à plusieurs heures.

D'autres élastomères appropriés incluent des compositions de diméthylpolysiloxane à deux composants qui sont catalysées par le platine à température ambiante ou à des températures élevées et susceptibles de réticulation par addition.

Les élastomères hydrophobes préférés pour une utilisation dans le dispositif selon l'invention incluent Silastic 382® et Silastic 4210® , tous deux commercialisés par Dow Corning.

La membrane de contrôle de la vitesse peut comprendre tout polymère biocompatible. Commodément, celle-ci comprendra le même polymère que la matrice de polymère.

La matrice de polymère et/ou la membrane de contrôle de la vitesse peuvent comprendre en outre un ou plusieurs matériaux de charge tels que la terre de diatomées ou la silice. En outre, la matrice de polymère peut comprendre un milieu de contraste aux rayons X tel que le sulfate de baryum, qui peut être utilisé à des fins d'identification.

Alors qu'il doit être évident que le dispositif intravaginal selon l'invention peut avoir toute forme et dimension compatibles avec une administration intravaginale, un dispositif préféré selon l'invention a la forme d'un anneau. Celui-ci comprend commodément un coeur central de matrice de polymère entouré par la membrane de contrôle de la vitesse.

Commodément, le diamètre global de l'anneau est dans la gamme de 52 à 62 mm, avec un diamètre de gaine (diamètre de section transversale de l'anneau) dans la gamme de 4 à 10 mm et un diamètre de coeur dans la gamme de 1 à 9 mm. Le diamètre de la membrane de contrôle de la vitesse est tel qu'il peut être fabriqué dans des tolérances acceptables par des méthodes connues dans la technique et se situe commodément dans la gamme de 1 à 4 mm, notamment de 2 à 3 mm. La géométrie de l'anneau peut être choisie selon la dose journalière d'oestrogène nécessaire et la durée du programme de traitement.

On pourra apprécier que la quantité d'oestrogène désirée qui est présente dans le dispositif sera fonction de l'oestrogène particulier à administrer ainsi que de l'affection à traiter. Des quantités commodes d'oestrogène présentes dans le dispositif sont de 1 à 50 % en poids, de préférence jusqu'à 15 % en poids, plus préférablement de 5 à 15 % en poids.

La quantité d'ester d'acide gras présente dans le dispositif sera également clairement fonction du principe actif particulier à délivrer ainsi que de l'affection à traiter. Commodément, la quantité d'ester d'acide gras présente dans le dispositif est de 1 à 50 %, de préférence de 5 à 20 % en poids. Il a été trouvé qu'on peut réaliser une gamme de doses d'oestrogène à partir d'anneaux de géométrie similaire en réglant le taux d'ester d'acide gras présent dans la matrice de polymère.

Le dispositif selon l'invention peut être utilisé pour traiter efficacement un certain nombre d'affections résultant d'un déficit en oestrogène, par ex. les symptômes vasomoteurs associés à un déficit en oestrogène, la vaginite atrophique, l'urétrite atrophique et l'ostéoporose associée à un déficit en oestrogène. Le dispositif est particulièrement efficace dans les thérapies substitutives hormonale et d'oestrogène. Comme le contrôle de la fertilité implique l'administration d'une quantité suffisante d'oestrogène pour empêcher l'ovulation, on pourra apprécier que le dispositif de délivrance de l'invention peut également être utilisé pour empêcher l'ovulation et agir ainsi comme un contraceptif efficace.

Les oestrogènes qui peuvent être délivrés par voie vaginale en utilisant le dispositif selon l'invention incluent l'oestrone et l'oestriol, en particulier le 17β-oestradiol qui est largement utilisé en thérapie hormonale substitutive (HRT). On peut obtenir des vitesses de libération journalières constantes jusqu'à 500 μg par jour pendant une période allant jusqu'à 12 mois, en utilisant un dispositif selon l'invention. La vitesse de libération d'oestrogène désirée est fonction de l'affection à traiter, mais peut varier sur une gamme de 10 à 200 μg par jour. Une dose de 17β-oestradiol physiologiquement efficace, suffisante pour traiter les symptômes post-ménopausiques, est considérée être de l'ordre d'au moins 50 μg par jour.

Les progestogènes pouvant être délivrés par voie vaginale en utilisant le dispositif selon l'invention incluent tous progestogènes connus pour être appropriés pour une utilisation en thérapie hormonale substitutive. Les progestogènes préférés incluent la progestérone, la médroxyprogestérone, la noréthistérone, la trimégestone et l'acétate de noréthistérone. Des quantités commodes de progestogène présentes dans le dispositif sont de 1 à 50 % en poids, de préférence jusqu'à 15 % en poids, plus préférablement de 5 à 15 % en poids.

Le progestogène et l'oestrogène pouvant être délivrés par voie vaginale en utilisant le dispositif selon l'invention incluent en particulier la trimégestone en association avec le 17β-oestradiol.

Les dispositifs de délivrance de médicaments par voie intravaginale selon l'invention peuvent être préparés par des méthodes bien connues dans la technique, telles que les techniques de moulage par injection, ou par extrusion d'un mélange de polymères. La géométrie des dispositifs peut varier grâce à l'utilisation de moules de taille appropriée ou de filières d'extrusion de taille appropriée.

Vue d'un autre aspect, l'invention fournit un procédé de fabrication d'un dispositif de délivrance de médicaments par voie intravaginale défini précédemment, ledit procédé comprenant les étapes consistant à combiner un agent actif avec un ester d'acide gras dans une matrice de polymère pour former un coeur ; et éventuellement à entourer ledit coeur d'une membrane de contrôle de la vitesse.

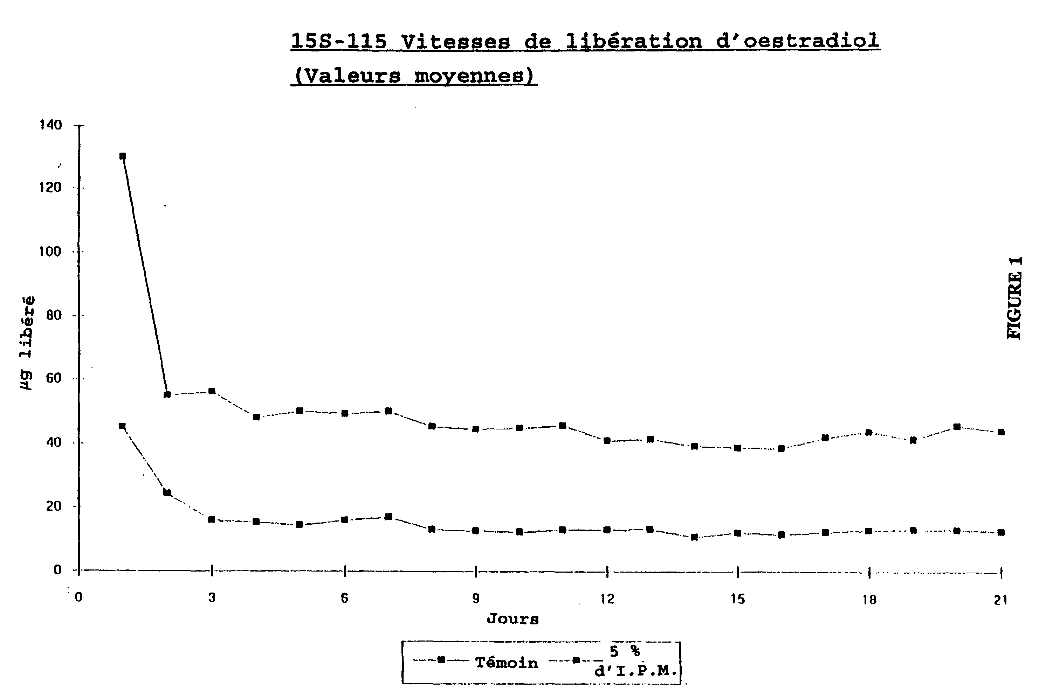

La présente invention sera maintenant décrite davantage à l'aide des exemples non limitatifs suivants et en référence aux Figures annexes 1 à 7 dans lesquelles, notamment les Figures 1 à 4 illustrent la vitesse de libération d'oestradiol à partir d'anneaux selon l'invention ayant un diamètre de coeur de 2 mm, un diamètre de gaine de 7,6 mm et une épaisseur de membrane de 2,8 mm.

Exemple 1

On a préparé un mélange de silicone en mélangeant 97 % en poids de polydiméthylsiloxane (Silastic 3099 disponible chez Dow Corning) avec 2,5 % en poids d'agent réticulant le n-propylorthosilicate. Le mélange résultant a ensuite été mélangé dans un rapport de 80 à 95 % en poids avec de 5 à 20 % en poids de myristate d'isopropyle (mélange 1). On a ensuite ajouté 5 % en poids de 17β-oestradiol et on a bien mélangé.

On a alors pesé des quantités appropriées du mélange ci-dessus et on a ajouté 0,5 % en poids du catalyseur l'octoate stanneux et on a mélangé pendant 1 minute. On a ensuite injecté le mélange final dans des moules de coeur de 2 mm et on l'a ultérieurement fait durcir à 80° C pendant 2 minutes.

Les coeurs actifs ont ensuite été enlevés des moules et placés dans un deuxième moule. On a ensuite formé une gaine autour du coeur par moulage par injection en deux étapes du mélange 1 avec 0,5 % en poids de catalyseur.

Les anneaux ont été soumis à un essai de libération in vitro dans 1 litre d'eau comme milieu de libération à 37° C.

La libération de 17β-oestradiol d'anneaux contenant 5 % en poids de myristate d'isopropyle sur la période d'essai de 21 jours est présentée à la Figure 1. On a obtenu une vitesse de libération d'ordre zéro de 40 μg par jour.

Exemple 2

Un deuxième jeu d'anneaux contenant 10 % en poids de myristate d'isopropyle a été produit comme à l'exemple 1.

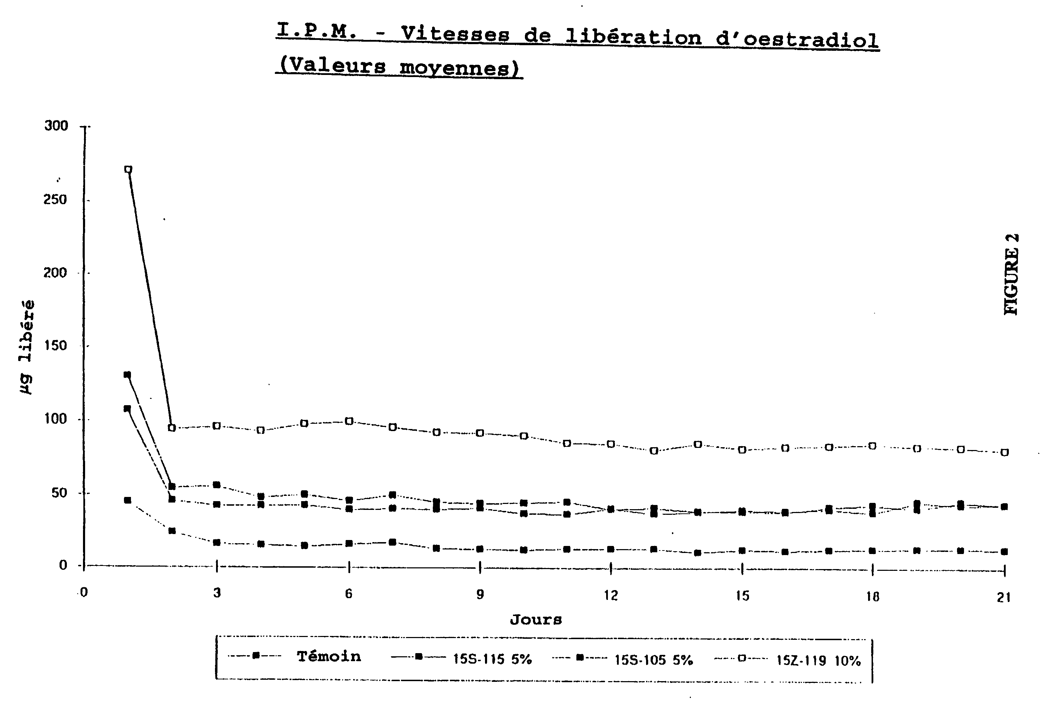

Les anneaux ont été soumis à un essai de libération in vitro dans 1 litre d'eau comme milieu de libération à 37° C. On peut voir à la Figure 2 la vitesse de libération de 17β-oestradiol à partir de ces anneaux. On a obtenu une vitesse de libération d'ordre zéro de 80 μg par jour.

Exemple 3

On a mélangé du Silastic 4210® (disponible chez Dow Corning) dans un rapport de 80 à 95 % en poids avec de 5 à 20 % en poids de myristate d'isopropyle (mélange 1). On a ensuite ajouté 5 % en poids de 17β-oestradiol et on a bien mélangé.

On a ensuite pesé 7 parties du mélange ci-dessus et on a ajouté 1 partie d'agent de durcissement et on a mélangé pendant 1 minute. Le mélange final a ensuite été injecté dans des moules de coeur de 2 mm et on l'a ultérieurement fait durcir à 80° C pendant 2 minutes.

Les coeurs actifs ont ensuite été enlevés des moules et placés dans un deuxième moule. On a ensuite formé une gaine autour du coeur par moulage par injection en deux étapes de 7 parties du mélange 1 et d'1 partie d'agent de durcissement.

Les anneaux ont été soumis à un essai de libération in vitro dans 1 litre d'eau comme milieu de libération à 37° C. On a obtenu une vitesse de libération d'ordre zéro de 60 μg de 17β-oestradiol par jour à partir d'anneaux contenant 10 % en poids de myristate d'isopropyle.

Exemple 4

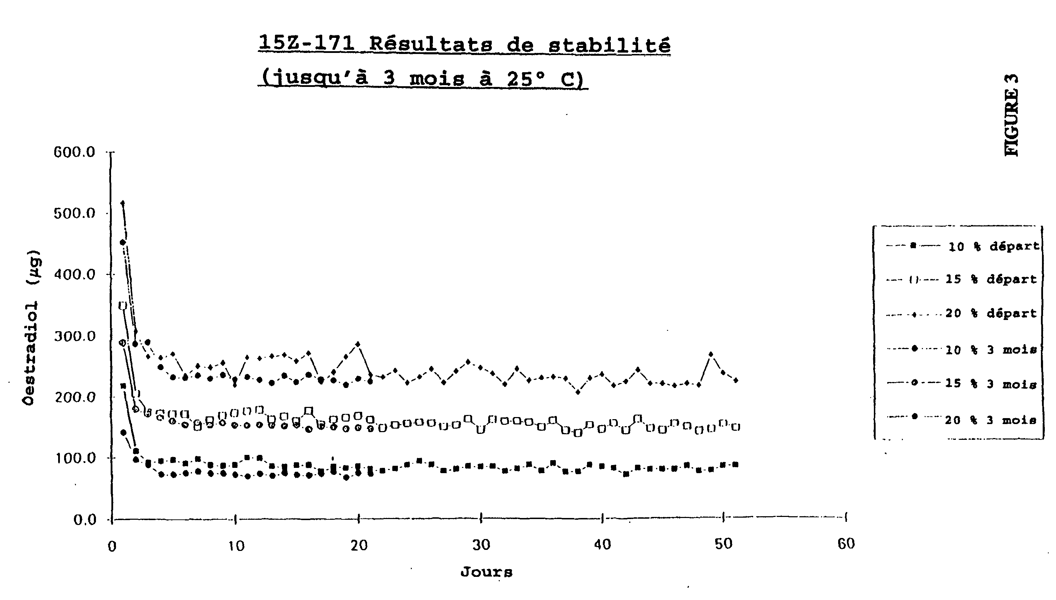

On a fabriqué des anneaux comme à l'Exemple 1 en utilisant 10, 15 et 20 % en poids de myristate d'isopropyle. Les anneaux ont été testés pour la libération sur 50 jours. On a obtenu des vitesses de libération d'ordre zéro de 80, 160 et 240 μg. Après 3 mois de stockage à 25°C, la libération est comme au départ (Figure 3).

Exemple 5

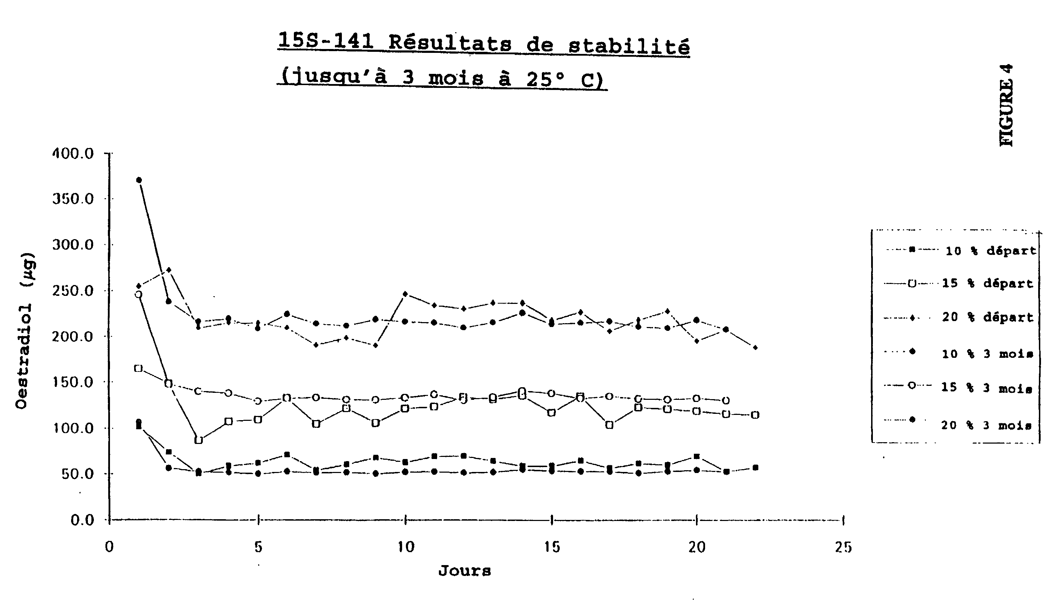

On a également fabriqué d'autres lots d'anneaux en utilisant le Silastic 4210 exactement comme à l'Exemple 3. La Figure 4 présente les résultats de l'essai de libération (au départ et après 3 mois de stockage à 25° C).

Exemple 6

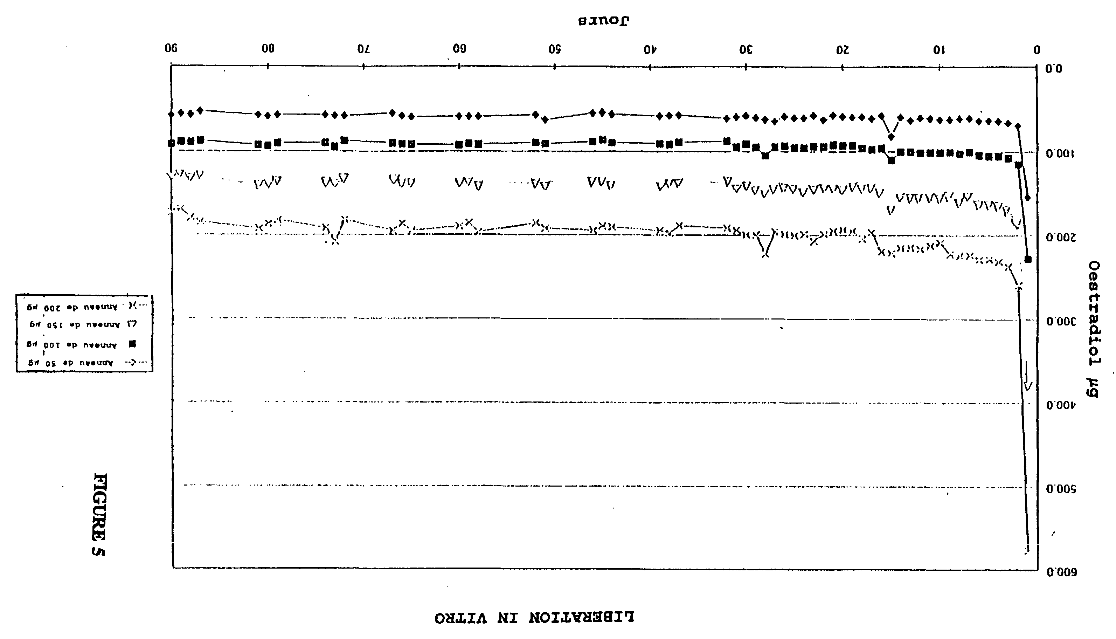

On a fabriqué des anneaux comme à l'Exemple 1 en utilisant 5 % en poids de 17β-oestradiol et 2,5 à 10 % en poids de myristate d'isopropyle. Une taille de coeur de 4,5 mm a été utilisée pour ces anneaux. Les anneaux ont été testés pour la libération et on a observé des vitesses de libération d'ordre zéro sur 90 jours (Figure 5).

Bien que l'invention ait été décrite en référence au 17β-oestradiol en particulier, elle s'étend également à des dispositifs de délivrance de médicaments par voie intravaginale pour l'administration d'autres principes actifs. De tels dispositifs peuvent être fabriqués par des procédés analogues à ceux décrits ci-dessus mais dans lesquels le 17β-oestradiol est remplacé par le principe actif nécessaire.

Exemple 7

Un mélange silicone a été préparé comme à l'exemple 1 en utilisant 10% en poids de myristate d'isopropyle (mélange 1). 5% en poids de mifépristone a ensuite été ajouté, et les coeurs actifs ont été fabriqués à partir de moules de 4,5 mm. On a ensuite formé une gaine autour du coeur par moulage par injection en deux étapes du mélange 1 comme à l'exemple 1.

La même procédure a été répétée sans ajout de myristate d'isopropyle dans la matrice silicone. Les anneaux ont été soumis à un essai de libération in vitro dans 1 litre d'eau comme milieu de libération à 37°C. On a obtenu une vitesse de libération d'ordre zéro respectivement de 400 μg par jour et de 100 μg par jour.

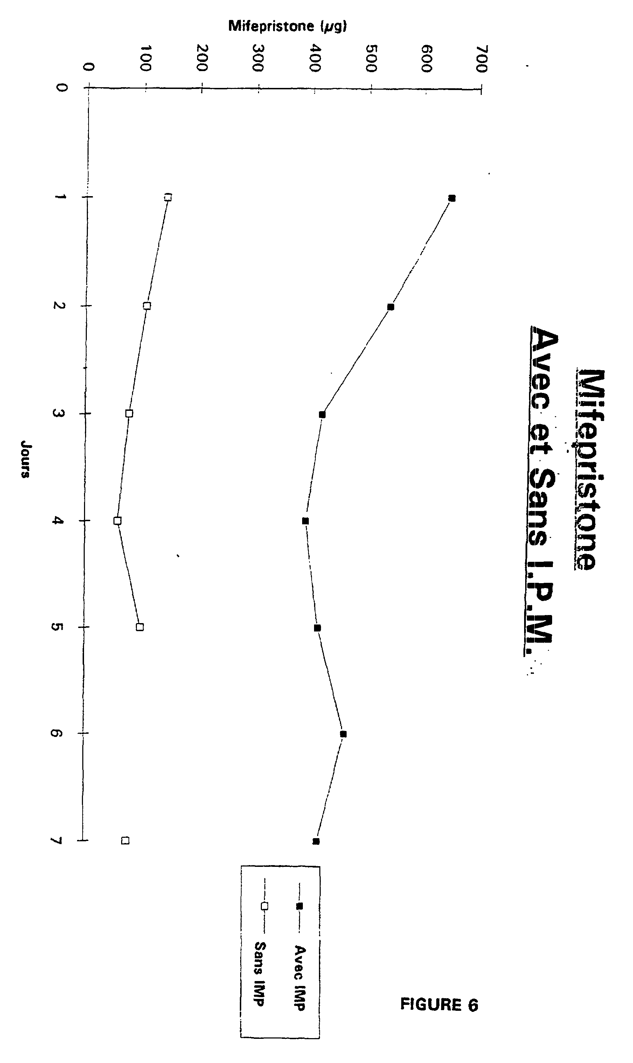

L'addition du myristate d'isopropyle a entraîné une multiplication par 4 de la vitesse de libération du principe actif tout en maintenant un schéma d'ordre zéro (figure 6).

Exemple 8

Un deuxième lot d'anneaux a été préparé de la même façon qu'à l'exemple 7, mais en utilisant comme principe actif la trimégestone. Les anneaux ont été soumis à un essai de libération in vitro.

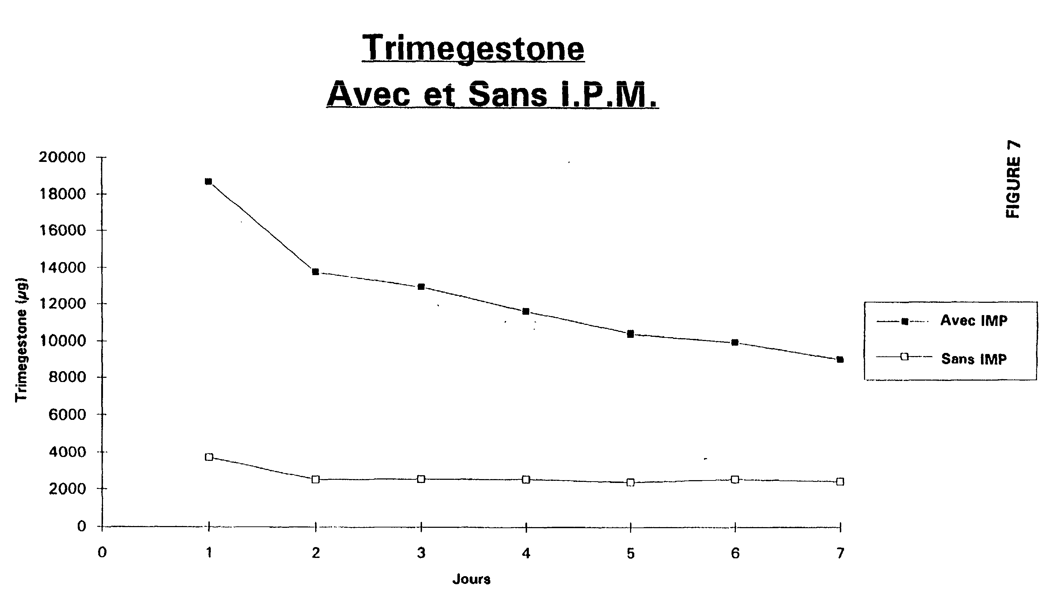

L'addition de myristate d'isopropyle a entraîné une multiplication par 5 de la vitesse de libération du principe actif, tout en maintenant un schéma d'ordre zéro (figure 7).

**Conclusion :**

Les exemples montrent que les esters d'acide gras permettent d'augmenter l'hydrophilicité de la matrice polymère, entraînent ainsi une augmentation de la solubilité des principes actifs dans la matrice et par conséquent améliorent la vitesse de libération du principe actif du dispositif.

Ceci permet une libération de doses thérapeutiques durables d'un grand nombre de molécules actives, sur de longues périodes dans le temps.

Ainsi, vue d'un aspect plus large, l'invention fournit un dispositif de délivrance de médicaments par voie intravaginale comprenant un principe actif dans une matrice de polymère, dans lequel la matrice de polymère comprend

EP 0 776 659 B1

en outre au moins un ester d'acide gras, de préférence contenant de 2 à 20 atomes de carbone.

D'autres principes actifs appropriés et leur utilisation thérapeutique sont énumérés à titre d'exemple dans le tableau suivant :

| ACTIF | CATEGORIE |
|---|---|
| Fluphénazine<br>Flupenthixol<br>Halopéridol | Antidépresseurs/Anxiolytiques |
| Buspirone<br>Alprazolam<br>Trifluoperzine | P.M.S. Anxiolytiques |
| Pyridoxine<br>Pridoxal<br>Pyridoxamine | P.M.S. Vitamine B6 |
| Cholécalciférol<br>Dihydrotachystérol<br>Ergocalciférol<br>Alfacalcidol | Vitamine D |
| d-Alpha Tocophérol | vitamine E |
| Clotrimazole<br>Enconazole<br>Iltraconazole | Antifongique |
| Buprénorfine<br>Lévorphanol<br>Phénopéridine<br>Fentanyl<br>Méthadone | Analgésiques opioïdes |
| Acide métanamique | Analgésiques non-opioïdes |
| Acyclovir<br>Vidarabine<br>Arildone<br>Idoxuridine | Antiviraux |
| Gestrinone | Endométriose |

**Revendications**

1.  Dispositif de délivrance de médicaments par voie intravaginale comprenant un agent actif dans une matrice de polymère, **caractérisé en ce que** ladite matrice de polymère comprend au moins un polyorganosiloxane et au moins un ester d'acide gras.

2.  Dispositif selon la revendication 1, **caractérisé en ce que** ledit ester d'acide gras est présent dans une quantité de 1 à 50 % en poids.

3.  Dispositif selon la revendication 1, **caractérisé en ce que** l'ester d'acide gras est présent dans une quantité de 5 à 20 % en poids.

4.  Dispositif selon l'une quelconque des revendications 1 à 3, **caractérisé en ce que** ledit ester d'acide gras est formé d'un acide gras comprenant de 2 à 20 atomes de carbone.

5.  Dispositif selon la revendication 4, **caractérisé en ce que** ledit acide gras est l'acide caproïque, laurique, myristique, oléique, linoléique, adipique ou lanolique.

**6.** Dispositif selon l'une quelconque des revendications précédentes, **caractérisé en ce que** ledit ester d'acide gras est formé d'un alcool contenant de 2 à 20 atomes de carbone.

**7.** Dispositif selon la revendication 6, **caractérisé en ce que** ledit alcool comprend de 2 à 4 atomes de carbone.

**8.** Dispositif selon l'une quelconque des revendications précédentes, **caractérisé en ce que** ledit ester d'acide gras est le myristate d'isopropyle.

**9.** Dispositif selon l'une quelconque des revendications précédentes comprenant un coeur central de matrice de polymère entouré d'une membrane de contrôle de la vitesse.

**10.** Dispositif selon l'une quelconque des revendications 1 à 9, **caractérisé en ce que** l'agent actif comprend un oestrogène et/ou un progestogène.

**11.** Dispositif selon l'une quelconque des revendications 1 à 10, comprenant un oestrogène et/ou un progestogène dans une quantité de 1 à 50% en poids.

**12.** Dispositif selon l'une quelconque des revendications 1 à 10, comprenant un oestrogène et/ou un progestogène dans une quantité allant jusqu'à 15% en poids.

**13.** Dispositif selon l'une quelconque des revendications 1 à 12, **caractérisé en ce que** ledit oestrogène est le 17β-oestradiol.

**14.** Dispositif selon l'une quelconque des revendications 1 à 12, **caractérisé en ce que** ledit progestogène est la progestérone, la médroxyprogestérone, la noréthistérone, la trimégestone ou l'acétate de noréthistérone.

**15.** Dispositif selon l'une quelconque des revendications 1 à 12, comprenant la trimégestone en association avec le 17β-oestradiol.

**16.** Dispositif selon la revendication 1, **caractérisé en ce que** l'agent actif comprend un antidépresseur-anxiolytique, un P.M.S. anxiolytique, un P.M.S. vitamine B6, une vitamine D, une vitamine E, un antifongique, un analgésique opioïde, un analgésique non opioïde, un antiviral ou un composé destiné à être utilisé dans le traitement de l'endométriose.

**17.** Dispositif selon les revendications 1 à 16, **caractérisé en ce que** l'agent actif est la fluphénazine, le flupenthixol, le halopéridol, la buspirone, l'alprazolam, la trifluoperzine, la pyridoxine, le pridoxal, la pyridoxamine, le choiécalciférol, le dihydrotachystérol, l'ergocalciférol, l'alfacalcidol, le d-alphatocophérol, le clotrimazole, l'enconazole, l'iltraconazole, la buprénorphine, le lévorphanol, la phénopéridine, le fentanyl, la méthadone, l'acide méfanamique, l'alcyclovir, la vidarabine, l'arildone, l'idoxuridine ou la gestrinone.

**18.** Procédé de fabrication d'un dispositif de délivrance de médicaments par voie intravaginale selon l'une quelconque des revendications précédentes, ledit procédé comprenant l'étape consistant à disperser un agent actif dans une matrice de polymère contenant au moins un ester d'acide gras.

**19.** Utilisation d'un dispositif selon l'une quelconque des revendications 10 à 15, comme contraceptif.

**20.** Utilisation d'un ester d'acide gras dans la fabrication d'un dispositif de délivrance de médicaments par voie intravaginale pour une libération d'ordre sensiblement zéro d'un agent actif chez une femelle humaine ou non humaine.

**Claims**

**1.** Device for delivering medicaments by intravaginal route comprising an active ingredient in a polymer matrix, **characterized in that** said polymer matrix comprises at least one polyorganosiloxane and at least one acid ester.

**2.** Device according to claim 1, **characterized in that** said fatty acid ester is present in a quantity of 1 to 50 % by weight.

**3.** Device according to claim 1, **characterized in that** the fatty acid ester is present in a quantity of 5 to 20 % by weight.

**4.** Device according to any one of claims 1 to 3, **characterized in that** said fatty acid ester is formed from a fatty acid comprising 2 to 20 carbon atoms.

**5.** Device according to claim 4, **characterized in that** said fatty acid is caproic, lauric, myristic, oleic, linoleic, adipic or lanolic acid.

**6.** Device according to any one of the previous claims, **characterized in that** said fatty acid ester is formed from an alcohol containing from 2 to 20 carbon atoms.

**7.** Device according to claim 6, **characterized in that** said alcohol comprises 2 to 4 carbon atoms.

**8.** Device according to any one of the previous claims, **characterized in that** said fatty acid ester is isopropyl myristate.

**9.** Device according to any one of the previous claims comprising a central core of the polymer matrix surrounded by a speed control membrane.

**10.** Device according to any one of claims 1 to 9, **characterized in that** the active ingredient comprises an estrogen and/or a progestogen.

**11.** Device according to any one of claims 1 to 10, comprising an estrogen and/or a progestogen in a quantity of 1 to 50 % by weight.

**12.** Device according to any one of claims 1 to 10, comprising an estrogen and/or a progestogen in a quantity ranging up to 15 % by weight.

**13.** Device according to any one of claims 1 to 12, **characterized in that** said estrogen is 17β-estradiol.

**14.** Device according to any one of claims 1 to 12, **characterized in that** said progestogen is progesterone, medroxyprogesterone, norethisterone, trimegestone or norethisterone acetate.

**15.** Device according to any one of claims 1 to 12, comprising trimegestone in combination with 17β-estradiol.

**16.** Device according to claim 1, **characterized in that** the active ingredient comprises an anxiolytic-antidepressant, an anxiolytic P.M.S., a vitamin B6 P.M.S., a vitamin D, vitamin E, an antifungal agent, an opioid analgesic, a non-opioid analgesic, an antiviral agent or a compound intended to be used in the treatment of endometriosis.

**17.** Device according to claims 1 to 16, **characterized in that** the active ingredient is fluphenazine, flupenthixol, haloperidol, buspirone, alprazolam, trifluoperazine, pyridoxine, pridoxal, pyridoxamine, cholecalciferol, dihydrotachysterol, ergocalciferol, alfacalcidol, d-alphatocopherol, clotrimazole, enconazole, iltraconazole, buprenorphine, levorphanol, phenoperidine, fentanyl, methadone, mefanamic acid, alcyclovir, vidarabine, arildone, idoxuridine or gestrinone.

**18.** Manufacturing process of a device for the delivery of medicaments by intravaginal route according to any one of the previous claims, said process comprising the stage consisting of dispersing an active ingredient in a polymer matrix containing at least one fatty acid ester.

**19.** Use of a device according to any one of claims 1 to 16, as a contraceptive.

**20.** Use of a fatty acid ester in the manufacture of a device for the delivery of medicaments by intravaginal route for a release of the order of approximately zero of an active ingredient in a human or non-human female.

**Patentansprüche**

**1.** Vorrichtung zur Zuführung von Arzneimitteln auf intravaginalem Weg, umfassend eine Wirksubstanz in einer Polymermatrix, **dadurch gekennzeichnet, daß** die Polymermatrix mindestens ein Polyorganosiloxan und mindestens einen Fettsäureester umfaßt.

**2.** Vorrichtung nach Anspruch 1, **dadurch gekennzeichnet, daß** der Fettsäureester in einer Menge von 1 bis 50 Gew.-% vorliegt.

**3.** Vorrichtung nach Anspruch 1, **dadurch gekennzeichnet, daß** der Fettsäureester in einer Menge von 5 bis 20 Gew.-% vorliegt.

**4.** Vorrichtung nach einem der Ansprüche 1 bis 3, **dadurch gekennzeichnet, daß** der Fettsäureester von einer Fettsäure gebildet wird, die 2 bis 20 Kohlenstoffatome umfaßt.

**5.** Vorrichtung nach Anspruch 4, **dadurch gekennzeichnet, daß** die Fettsäure Capronsäure, Laurinsäure, Myristinsäure, Ölsäure, Linolsäure, Adipinsäure oder Lanolinsäure ist.

**6.** Vorrichtung nach einem der vorangehenden Ansprüche, **dadurch gekennzeichnet, daß** der Fettsäureester von einem Alkohol gebildet wird, der 2 bis 20 Kohlenstoffatome enthält.

**7.** Vorrichtung nach Anspruch 6, **dadurch gekennzeichnet, daß** der Alkohol 2 bis 4 Kohlenstoffatome umfaßt.

**8.** Vorrichtung nach einem der vorangehenden Ansprüche, **dadurch gekennzeichnet, daß** der Fettsäureester Myristinsäureisopropylester ist.

**9.** Vorrichtung nach einem der vorangehenden Ansprüche, die einen Mittelkern aus Polymermatrix umfaßt, der mit einer Membran zur Kontrolle der Geschwindigkeit umgeben ist.

**10.** Vorrichtung nach einem der Ansprüche 1 bis 5, **dadurch gekennzeichnet, daß** die Wirksubstanz ein Östrogen und/oder ein Progestagen umfaßt.

**11.** Vorrichtung nach einem der Ansprüche 1 bis 10, die ein Östrogen und/oder ein Progestagen in einer Menge von 1 bis 50 Gew.-% umfaßt.

**12.** Vorrichtung nach einem der Ansprüche 1 bis 10, die ein Östrogen und/oder ein Progestagen in einer Menge bis zu 15 Gew.-% umfaßt.

**13.** Vorrichtung nach einem der Ansprüche 1 bis 12, **dadurch gekennzeichnet, daß** das Östrogen 17β-Estradiol ist.

**14.** Vorrichtung nach einem der Ansprüche 1 bis 12, **dadurch gekennzeichnet, daß** das Progestagen Progesteron, Medroxyprogesteron, Norethisteron, Trimegeston oder Norethisteronacetat ist.

**15.** Vorrichtung nach einem der Ansprüche 1 bis 12, die Trimegeston in Kombination mit 17β-Estradiol umfaßt.

**16.** Vorrichtung nach Anspruch 1, **dadurch gekennzeichnet, daß** die Wirksubstanz ein Antidepressivum-Anxiolytikum, ein PMS-Anxiolytikum, ein PMS-Vitamin $B_6$, Vitamin D, Vitamin E, ein antifungales Mittel, ein Opioid-Analgetikum, ein Nicht-Opioid-Analgetikum, ein Antivirusmittel oder eine Verbindung, die dazu bestimmt ist, bei Behandlung von Edometriose verwendet zu werden, umfaßt.

**17.** Vorrichtung nach den Ansprüchen 1 bis 16, **dadurch gekennzeichnet, daß** die Wirksubstanz Fluphenazin, Flupenthixol, Haloperidol, Buspiron, Alprazolam, Trifluoperazin, Pyridoxin, Pridoxal, Pyridoxamin, Cholecalciferol, Dihydrotachysterol, Ergocalciferol, α-Calcidol, d-α-Tocopherol, Clotrimazol, Enconazol, Iltraconazol, Buprenorphin, Levorphanol, Phenoperidin, Fentanyl, Methadon, Mefanamsäure, Alcyclovir, Vidarabin, Arildon, Idoxuridin oder Gestrinon ist.

**18.** Verfahren zur Herstellung einer Vorrichtung zur Zuführung von Arzneimitteln auf intravaginalem Weg nach einem der vorangehenden Ansprüche, wobei das Verfahren die Stufe umfaßt, die im Dispergieren einer Wirksubstanz in einer Polymermatrix, welche mindestens einen Fettsäureester enthält, besteht.

**19.** Verwendung einer Vorrichtung nach einem der Ansprüche 10 bis 15 als Kontrazeptivum.

**20.** Verwendung eines Fettsäureester bei der Herstellung einer Vorrichtung zur Zuführung von Arzneimitteln auf intravaginalem Weg zur Freisetzung einer Wirksubstanz in der Größenordnung von nahe Null bei einer Frau oder einem nicht-humanen Weibchen.

15S-115 Vitesses de libération d'oestradiol
(Valeurs moyennes)

FIGURE 1

EP 0 776 659 B1

12

EP 0 776 659 B1

13

FIGURE 2

I.P.M. - Vitesses de libération d'oestradiol
(Valeurs moyennes)

Jours

Témoin ----■---- 155-115 5% ----■---- 155-105 5% ----□---- 152-119 10%

μg libéré

15Z-171 Résultats de stabilité
(jusqu'à 3 mois à 25° C)

FIGURE 3

EP 0 776 659 B1

14

Légende:
— ■ — 10 % départ
— ⊓ — 15 % départ
— ♦ — 20 % départ
···●··· 10 % 3 mois
···○··· 15 % 3 mois
···●··· 20 % 3 mois

Oestradiol (µg)

Jours

15S-141 Résultats de stabilité
(jusqu'à 3 mois à 25° C)

FIGURE 4

EP 0 776 659 B1

15

FIGURE 5

**Mifepristone Avec et Sans I.P.M.**

Mifepristone (µg) vs Jours

Légende:
- Avec IMP
- Sans IMP

**FIGURE 6**

FIGURE 7